# EUROPEAN PATENT SPECIFICATION

(11) **EP 2 889 021 B1**
(45) Date of publication and mention of the grant of the patent: **13.12.2017**
(21) Application number: 14197293.5
(22) Date of filing: 11.12.2014
(51) Int. Cl.: A61F 2/954, A61F 2/958

(54) **Prewire channel stent**
Vorverdrahteter Kanalstent
Endoprothèse de canal pré-câblé

(30) Priority: 29.12.2013 CN 201320895986 U
(43) Date of publication of application: 01.07.2015
(73) Proprietor: Tang, Lilong, Xiangtan, Hunan Province 411100 (CN)
(72) Inventor: Tang, Lilong, Xiangtan, Hunan Province 411100 (CN)
(74) Representative: Zahn, Matthias

(56) References cited:
- EP-A1- 0 897 700
- DE-U1- 29 708 803
- US-A- 6 007 517
- US-A1- 2004 143 286
- US-A1- 2012 089 220

## Description

### Field of the Invention

The present invention relates generally to a medical device for the treatment of vascular disease.

### Background

Cardiovascular stents are widely used in the treatment of vascular diseases. For example, stents have been broadly implanted in vessels to treat vessel narrowing, occlusion, aneurysm, and dissection, among other conditions. A typical conventional or known stent contains a metallic mesh material that is mounted or carried on a deflated balloon. In the centre of the balloon is a channel, through which a guidewire can pass to facilitate movement through the arteries; however, there is no channel between the metallic mesh material and the balloon. A guidewire is inserted to the vessel and past the location that needs to be treated and then the cardiovascular stent mounted or carried on a deflated balloon is inserted into the vessel and delivered to the desired location through this guidewire to facilitate movement through the arteries. When the stent is at the desired location, the balloon is inflated to expand and, thereby, deploy the stent to support the artery.

Vessels have branches; when narrowing, these narrowed branches are referred to as "bifurcation lesions". When treating a bifurcation lesion with a conventional or known stent, one guidewire is delivered to the main branch, and another guidewire is delivered to a side branch. These two wires have to be exchanged (which is called wire-exchange) during the operation. This procedure is complicated and time-consuming.

In addition, when an ostial lesion (usually defined as a lesion within 3 mm of the ostium of the vessel at the aorto-ostial or branch-ostial junction) is treated, a typical conventional or known stent has to be deployed precisely at the desired location. To ensure precise deployment at the desired location, some operators like to employ the Szabo technique: specifically, they inflate part of the balloon with low pressure, and then deflate the balloon premounted by metallic mesh material to allow the guidewire to pass through the space between the metallic mesh material and the balloon for the precise deployment of the stent. However, this procedure has a risk of stent dislodgement. Details for the Szabo technique can be found in the following paper: "Jain RK, Padmanabhan TN, Chitnis N. Causes of failure with Szabo technique - an analysis of nine cases. Indian Heart J. 2013 May-Jun; 65(3):264-8. doi: 10.1016/j.ihj.2013.04.023. Epub 2013 Apr 10", referred to as "Jain et al."

US Patent No. 8,771,342 issued July 8, 2014 to Vardi is of interest for showing the background of this type of invention. In this reference, a method of deploying a stent in a bifurcation is shown that includes introducing two guidewires through the main vessel and using the two guidewires for guiding a dual lumen catheter carrying a stent first to an initial position proximal to the stent deployment position, retracting one wire, and projecting it from the catheter and through a side of the stent a branch guidewire into the second branch vessel, and then moving the catheter to the predetermined deployment position while guided by the main guidewire in the first branch vessel, and the branch guidewire in the second branch vessel. The stent is then expanded, and the catheter is removed with the stent remaining in its deployed position An alternative embodiment includes introducing one wire, advancing the system over the one wire and subsequently introducing the second wire.

US Patent No. 7,771,462 issued August 10, 2010 to Davidson, et al. is of interest for showing the background of this type of invention. This reference describes a designed catheter system comprises a catheter having a catheter body with a distal end, a proximal end, a main vessel guidewire lumen for receiving a main vessel guidewire and a balloon disposing at the distal end of the catheter body. The catheter further includes a side member that is disposed adjacent to the catheter body. The side member has a distal end, a proximal end, and a branch vessel guidewire lumen for receiving a branch vessel guidewire. A stent having a side hole is disposed over the balloon, and a distal portion of the side member is disposed beneath at least a portion of the stent while being adjacent to and movable with respect to the balloon.

US Patent No. 8,828,071 issued September 9, 2014 to Bourang et al is of interest for showing the background of this type of invention. This references discusses a system for treating a bifurcation that includes first and second delivery catheters, each having an expandable member. A stent having a side hole is disposed on the second delivery catheter. A portion of the first delivery catheter is disposed under a portion of the stent. The first delivery catheter is slidable relative to the second delivery catheter, and the first delivery catheter passes through the side hole. Expansion of the first expandable member expands a proximal portion of the stent in a main branch vessel, and expansion of the second expandable member expands a distal portion of the stent in a side branch vessel.

US Patent No. 8,808,347 issued August 19, 2014 to Bourang et al is of interest for showing alignment of the side branch stent with the main branch stent. This reference describes a system for treating a bifurcation includes a first radially expandable stent and a second radially expandable stent. The first stent has a side hole and a plurality of lateral elements extending from the side hole. The second stent has a plurality of axial elements extending away from the proximal end of the second stent. The axial elements of the second stent interdigitate with the lateral elements of the first stent when both stents have been expanded.

US Patent No. 8,795,347 issued August 5, 2014 to Bourang et al is of interest for showing methods and systems for treating a bifurcation with provisional side branch stenting.

US Patent Application 2012 / 0089220 A1 to Lualdi discloses a device for delivering and positioning an endolumenal expandable prosthesis for a bifurcation. There is guidewire tracking with a single guidewire lumen disposed within a wall structure.

There is a need for a newly designed stent to overcome the shortcomings of the typical conventional or known stent.

### Summary

From the foregoing, it is seen that it is a problem in the art to provide a device meeting the above requirements. According to the present invention, a device is provided which meets the aforementioned requirements and needs in the prior art. Specifically, the device according to the present invention provides a prewire channel stent having a structure that includes a balloon having a metallic mesh material outside the balloon, a central passage with ancillary components that inflate or deflate the balloon, and a second passage disposed between the metallic mesh material and the balloon.

Other objects and advantages of the present invention will be more readily apparent from the following detailed description when read in conjunction with the accompanying drawings. The invention is defined by claim 1.

### Brief Description of the Drawings

FIG. 1 is a top elevational view of a prewire channel stent in which a balloon is in an inflated status.
FIG. 2 is a top elevational view of the prewire channel stent of FIG. 1 in which the balloon is in a deflated status.
FIG. 3A is a transverse cross-sectional view of the prewire channel stent of FIG. 1 in which the balloon is in the inflated status.
FiG. 3B is a partial transverse cross-sectional view of the prewire channel stent of FIG. 1 in which the balloon is in the inflated status, but wherein the channel has an additional wall.
FIG. 4 is a longitudinal cross-sectional view of the prewire channel stent of FIG. 1, wherein the balloon is in the inflated status.
FIG. 5 is a transverse cross-sectional view of the prewire channel stent of FIG. 2 in which the balloon is in the deflated status.
FIG.6 is a a longitudinal cross-sectional view of the prewire channel stent of FIG. 1, wherein the balloon is in the inflated status and a guidewire is in a lumen of a catheter, and another guidewire is in the channel or space.

### Detailed Description

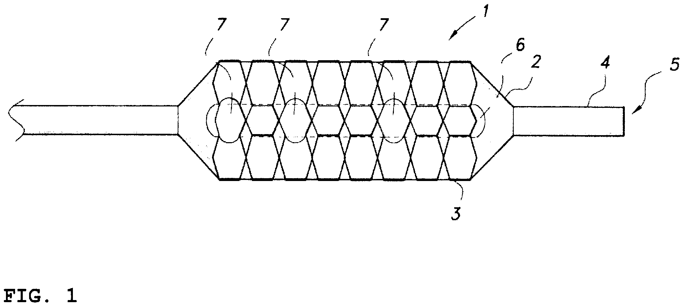

FIG. 1 is a top elevational view of a prewire channel stent 1 having an expandable balloon 2, and in which the expandable balloon 2 is in an inflated status. This expandable balloon 2 is in the inflated status during deployment, and is in a deflated status before deployment. A metallic mesh material 3 is premounted or carried on the expandable balloon 2.

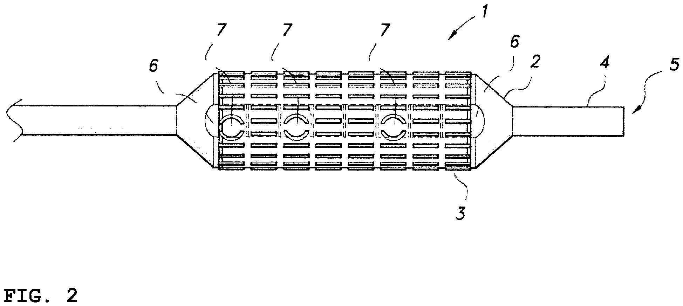

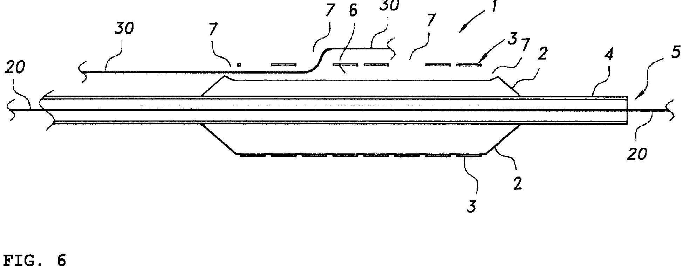

The metallic mesh material 3 is expanded when the balloon 2 is inflated during deployment, but is in a systolical status when the balloon 2 is deflated (i.e. is in its deflated status) before deployment (FIG. 2). A central catheter 4 goes through the balloon 2, and has a lumen 5 which allows a tracking guidewire 20 (shown in FIG. 6) to go therethrough.

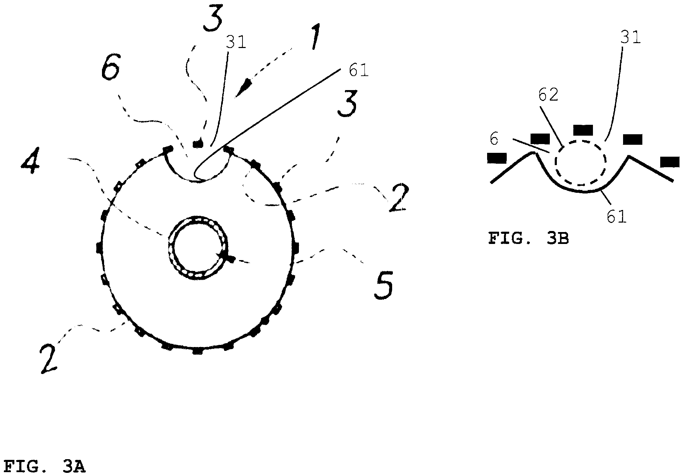

A channel 6 is shown in dashed outline in FIG. 1 and FIG. 2, and is provided between the metallic mesh material 3 and the expandable balloon 2. Channel 6 can also be regarded as a space (between metallic mesh material 3 and expandable balloon 2). A "channel" is considered to have a wall as boundary. An embodiment with a dedicated wall will be explained in connection with FIG. 3B.

A plurality of openings 7 (shown in FIGS. 1, 2, 4, and 6) are provided for communication between the channel 6 and a region outside the metallic mesh material 3. The openings 7 are formed through the metallic mesh material 3 as shown in FIG. 6. The openings 7 are provided so that a channel guidewire 30 can pass into the channel 6 and then exit through one of the openings 7, as shown in FIG. 6.

### Alternative Embodiments and Variations

Alternative or additional embodiments are contemplated as being within the scope of the present invention. In one such embodiment, the channel 6 can have a lining, for example a tubular lining which could be folded balloon or other material, through which the wire 30 can pass. In this embodiment, the lining would include openings that correspond to the openings 7. In another such embodiment, the channel 6 can be limited in extent such that is passes only partway along the length of the metallic mesh material 3; in a further variation on this embodiment, it is contemplated that fewer openings 7 could be provided than the number shown. Also, the number of openings 7 is not limited to the number shown and illustrated; more or fewer such openings 7 could be provided, within the scope of the present invention.

In use, one or more guidewires 30 can go through one of the openings 7 and into the channel 6 when the metallic mesh material 3 is in the systole status and the balloon 2 is in the deflated status. The channel 6 can be substantially closed or - alternatively - can remain open until such time as the metallic mesh material 3 is in the diastolic status and the balloon 2 is in the inflated status.

As seen in FIG. 1, the balloon 2 extends longitudinally along the central catheter 4. The metallic mesh material 3 shown in FIG. 1 surrounds the central region of the balloon 2. The channel 6 has the openings 7 disposed as shown, such that the guidewire 30 can enter and leave the channel 6 through any two of these openings 7. FIG. 6 illustrates this.

FIG. 2 is a top elevational view of the prewire channel stent 1 of FIG. 1 in which the balloon 2 is in a deflated status. The channel 6 has the openings 7 disposed at one and another end and also along the middle of the channel 6 as shown in FIG. 2, such that a guidewire can enter and leave the channel 6 at the openings 7. The channel 6 is shown in dashed outline in FIG. 1 and in FIG. 2, and is provided between the metallic mesh material 3 and the balloon 2.

FIG. 3A is a transverse cross-sectional view of the prewire channel stent 1 of FIG. 1 in which the balloon 2 is in the inflated status. The curved surface of the balloon 2 is visible and bounds a lower side 61 of the channel 6. The metallic mesh material 3 bounds an upper side 31 of the channel 6. The channel 6 can be substantially closed, or alternatively can still remain open, when the metallic mesh material 3 is in the diastolic status and the balloon 2 is in the inflated status. The terms "upper" and "lower" are conveniently introduced for clarity of explanation, but in implementations the stent 1 can be located differently.

FIG. 3B is a partial transverse cross-sectional view of the prewire channel stent of FIG. 1 in which the balloon is in the inflated status, wherein channel 6 has an additional wall 62. In other words, the prewire channel stent 1 has the second passage (i.e., channel 6) implemented with wall 62 located between the metallic mesh material 3 and the balloon 2. The wall 62 could exist not only in the inflated status but also in deflated status.

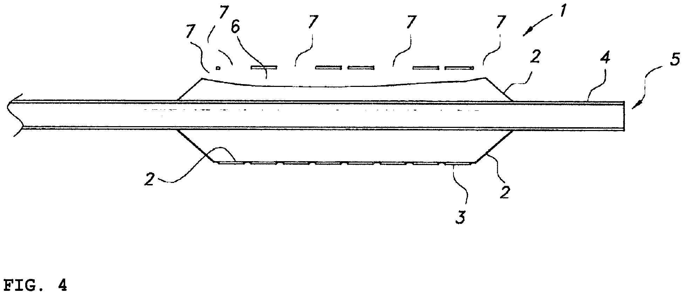

FIG. 4 is a longitudinal cross-sectional view of the prewire channel stent 1 of FIG. 1, wherein the balloon 2 is in the inflated status. This view shows a side view of the channel 6. In this view, the curved surface of the balloon 2 is visible and bounds the lower side of the channel 6. Also as shown in this view, the metallic mesh material 3 bounds the upper side of the channel 6. The channel 6 can exist or, alternatively can disappear (i.e. collapse) when the balloon 2 is in the inflated status. Openings 7 exist at both ends of the channel 6. More openings can exist between the both ends of the channel 6, as shown in the drawings. The channel 6 can extend from the end of metallic mesh material to the another end of metallic mesh material.

The terminology "channel" or "space", as used herein, means that the passageway is formed by the element referred to as the "channel 6", and this passageway can be in the form of a channel formed in the adjacent materials or can be provided as a space between the adjacent materials. The width of the channel 6 is selected to be sufficient for passage of the wire 30 therethrough in the manner explained above, and can be relatively thin and narrow or can be relative wide. All such variations are contemplated as being within the scope of the present invention.

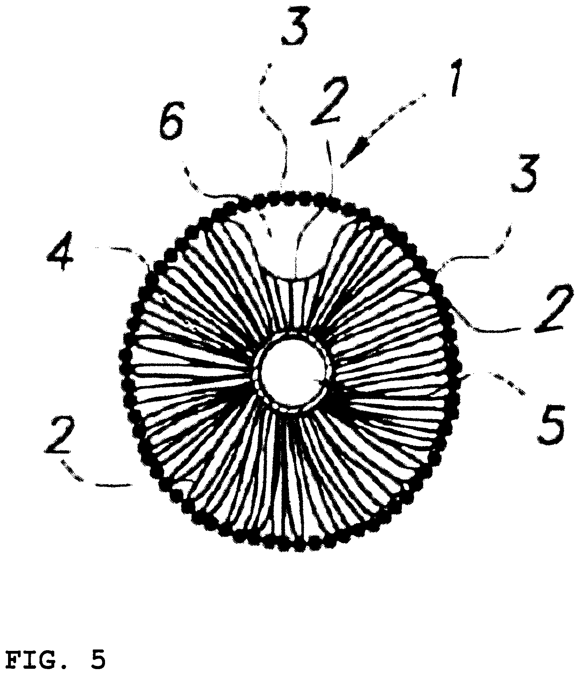

FIG. 5 is a transverse cross-sectional view of the prewire channel stent 1 of FIG. 2 in which the balloon 2 is in the deflated status. The parts shown in this view are those described hereinabove. In this view, the folds of the balloon 2 are visible. The upper boundary is the metallic mesh material. The channel 6 is shown as being relatively large in this view for the sake of clarity, and is not limited to this specific size or shape shown.

FIG. 6 is a longitudinal cross-sectional view of the prewire channel stent 1 of FIG. 1, wherein the balloon 2 is in the inflated status. This view shows a side view of the channel 6 with guidewire 30 going through the channel 6 and the guidewire 20 passing through the lumen 5 of the catheter 4. The guidewire 30 can go through any openings 7 of the channel 6. The channel 6 can be substantially closed, or alternatively can still remain open, when the metallic mesh material 3 is in the diastolic status and the balloon 2 is in the inflated status.

In the above discussion, a guidewire 20 is mentioned. The role of the guidewire 20 is for tracking. When treating the patients, the guidewire 20 is firstly sent to the vessels which are to be treated. Thereafter, a balloon and stent are sent through the guidewire 20 (by tracking) to the desired position of the vessels. All known conventional stents and balloons currently designed have a central channel (corresponding to channel 4 of the present invention) with a lumen for the guidewire 20 to go through. However, there is not any channel or space in currently designed stents (referred to herein as "conventional stents") that correspond to the channel 6 of the present invention which is for another guidewire (i.e., a second guidewire, namely guidewire 30 shown in FIG. 6) between the metallic mesh material 3 and the balloon 2.

Vessels in the human body can be described somewhat like highways in the country which have forks or branches (i.e., bifurcation). In a patient, therefore, if the narrow position is in bifurcation, it is necessary to use two guidewires in each of the branches. So when the stent is sent through the lumen 5 of its central channel 4 by one guidewire in a bifurcation position to release the stent in main branch, then another guidewire in the side branch must be between the metallic mesh material and the vessel wall (in other words, the wire is outside the stent). The guidewire outside the stent in the side branch should be pulled back and then sent to the main branch again through the inside stent and the guidewire in original main branches should be pulled back and sent to side branch across the metallic mesh material through the inside stent (or the doctor can send another wire across the metallic mesh material to the side branch through inside the stent). This process is called "wire exchange" which is achieved in the vessels and therefore is complicated and time consuming and sometimes cannot be finished because of the complications that may arise. In the present invention, the channel 6 is provided so that the guidewire in the side branch can go across the metallic mesh material through the inside of the stent outside the human body. Therefore, wire exchange is not necessary (i.e. this makes the step of wire exchange unnecessary).

When an ostial lesion is treated, a typical conventional or known stent has to be deployed precisely at the desired location. To ensure precise deployment at the desired location, some operators like to employ the Szabo technique (cf. Jain et al.): specifically, they inflate part of the balloon with low pressure, and then deflate the balloon to allow the guidewire to pass through the space between the metallic mesh material and the balloon. However, this procedure has a risk of stent dislodgement (cf. Jain et al.). In the present invention, the channel 6 is provided to avoid the risk of stent dislodgement.

The lesions involved in the bifurcation of the vessels could have different lengths. The principle of treating such lesions is to use the stent to fully cover the lesions. The channel 6 can be a different length from one end of the metallic mesh material 3 of the stent to the another end of metallic mesh material 3. The openings 7 of the channel 6 can be provided in multiple locations provided along the channel 6. This design allows the guidewire 30 to go across any openings 7 of the channel 6 based on the need of the length of the lesion in the vessel.

This stent system can be applied not only in vessels, but also in bronchi, bile ducts, urethrae, esophagi, and other organs or tissues.

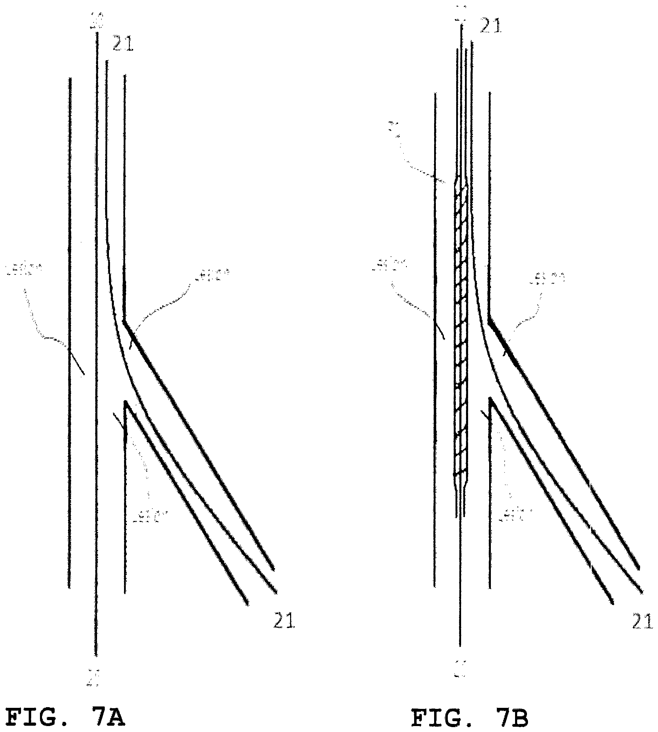

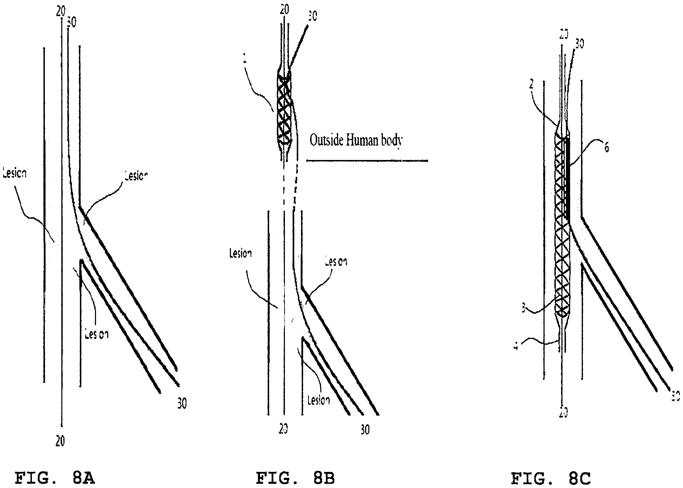

The following illustrates a working example for a conventional stent, shown in FIGS. 7A - 7E, and a working example for the Pre-Wire Channel Stent of the present invention, shown in FIGS. 8A - 8E.

The procedures of a conventional or known stent implant for bifurcation lesion in a vessel as explained as follows. Step 1 is shown in FIG. 7A, wherein a guidewire 20 is sent to a main branch and a guidewire 21 is sent to a side branch.

Step 2 is shown in FIG. 7B, wherein a system P1 of a conventional or known stent and balloon is sent through the guidewire 20 (by tracking) to the desired position of the vessels.

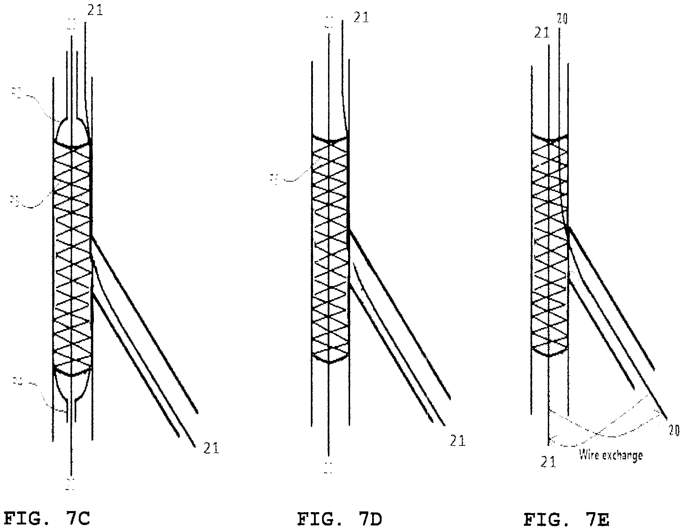

Step 3 is shown in FIG. 7C, a balloon P2 is inflated and a conventional stent P3 is dilated to support the vessel, and the guidewire 20 is still in the main branches through the lumen of the stent and the guidewire 21 is in the side branch through the space between the vessel wall and the metallic mesh material of the stent (i.e., outside the stent).

Step 4 is shown in FIG. 7D, wherein the balloon P2 is deflated and pulled out of the vessel. The guidewire 20 is still in the main branches through the lumen of the stent and the guidewire 21 is in the side branch through the space between the vessel wall and the metallic mesh material of the stent (i.e., outside the stent).

Step 5 is shown in FIG. 7E, in which the guidewire 20 is pulled back and then sent in the lumen of the stent through the metallic mesh to the side branch. The guidewire 21 is also pulled back and then sent through the lumen of the stent to main branch. The process of " wire exchange" is finished, as schematically indicated by the dashed lines in FIG. 7E.

The following illustrates the procedures for use of the prewire channel stent implant 1 of the present invention for a bifurcation lesion in a vessel. Step 1 is shown in FIG. 8A, wherein the guidewire 20 is sent to the main branch and the guidewire 30 is sent to the side branch.

Step 2 is shown in FIG. 8B, wherein the prewire channel stent 1 with the guidewire 30 goes through the channel 6 while outside the human body and is then sent through the guidewire 20 (by tracking) to the desired position of the vessels.

Step 3 is shown in FIG. 8C, wherein the prewire channel stent 1 with the guidewire 30 goes through the channel 6 is sent by tracking guidewire 20 to the desired position of the vessel (at the bifurcation lesion). The balloon 2 is in the deflated status in this view, and the metallic mesh material 3 is mounted in the balloon 2 and the guidewire 30 in the lumen of the catheter 4.

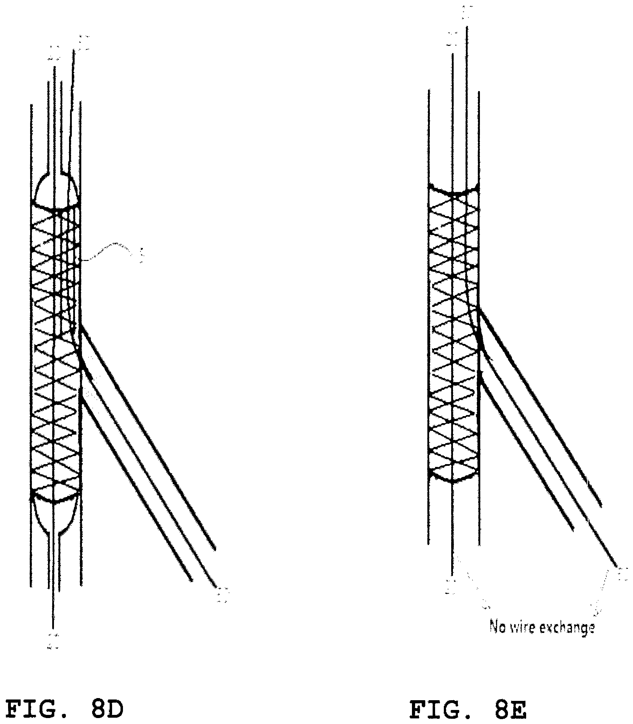

Step 4 is shown in FIG. 8D, wherein the balloon 2 is inflated and the metallic mesh material 3 is dilated. The guidewire 30 from the side branch is in the channel 6 and the guidewire 20 is disposed in the main branch.

Step 5 is shown in FIG. 8E, wherein the balloon 2 is deflated and withdrawn from the vessel. As seen in FIG. 8E, "wire exchange" is not necessary. This is in contrast to FIG. 7E of the prior device.

### References

- 2: balloon
- 3: mesh material
- 4: central catheter / first passage
- 6: channel / second passage
- 7: opennings
- 20: guidewire
- 21: guidewire
- 30: guidewire
- 31: upper side
- 61: lower side

## Claims

1. A medical device having:
a central catheter (4) with a lumen (5), said central catheter (4) having the lumen (5) as a central passage therethrough;
a balloon (2) disposed outside said central catheter (4), the balloon being inflatable and deflatable, with an inflated status during deployment, with an deflated status before deployment;
a metallic mesh material (3) disposed outside said balloon (2) and surrounding said balloon (2),
the medical device having a channel (6) disposed between said metallic mesh material (3) and said balloon (2), said channel (6) being adapted to receive a channel guidewire (30),
the medical device **characterized in that** the mesh material (3) is premounted on the inflatable balloon, wherein the mesh material (3) is to be expanded when the balloon (2) is inflated during deployment, and **in that** openings (7) through the metallic mesh material (3) are provided in the channel (6) at multiple locations along the channel (6) so that the channel guidewire (30) can pass into the channel (6) and exit through one of the openings (7), wherein
the channel (6) has an additional wall (62) located between the metallic mesh material (3) and the balloon (2), and
the channel has a first boundary by the balloon being folded and has a second boundary by the metallic mesh material (3).

2. The medical device according to claim 1, wherein the channel (6) is limited in extent such that it passes only partway along the length of the metallic mesh material (3).

3. The medical device according to claim 2, having a plurality of passages between the balloon (2) and the metallic mesh material (3).

## Patentansprüche

1. Ein medizinisches Gerät, enthaltend:
einen Zentralkatheter (4) mit einem Lumen (5), der Zentralkatheter (4) das Lumen (5) als eine zentrale Passage aufweisend;
einen Ballon (2), der außerhalb des Zentralkatheters (4) angeordnet ist, der Ballon aufblasbar und zusammenziehbar, im aufgeblasenen Zustand während des Hineinbringens, im zusammengezogenen Zustand vor dem Hineinbringen;
ein metallisches Maschen-Material (3), das außerhalb des Ballons (2) angeordnet ist, und das den genannten Ballon (2) umgibt,
das medizinische Gerät mit einem Kanal (6), der zwischen dem metallisches Maschen-Material (3) und dem Ballon (2) angeordnet ist, wobei der Kanal (6) zum Aufnehmen eines Kanal-Führungsdrahtes (30) angepaßt ist;
das medizinische Gerät **dadurch gekennzeichnet, daß** das metallisches Maschen-Material auf dem aufblasbaren Ballon vormontiert ist, wobei das metallische Maschen-Material (3) zum Expandieren vorgesehen ist, wenn der Ballon (2) während des Hineinbringens aufgeblasen wird, und **dadurch gekennzeichnet, daß** Öffnungen (7) durch das metallische Maschen-Material (3) at mehreren Stellen vorgesehen sind, so daß der Kanal-Führungsdraht (30) in den Kanal (6) eintreten kann und durch eine der Öffnungen (7) austreten kann, wobei
der Kanal (6) eine zusätzliche Wand enthält, die sich zwischen dem metallisches Maschen-Material (3) und dem Ballon (2) befindet, und
der Kanal eine erste Begrenzung hat, die durch den Ballon gebildet wird, der gefaltet ist, und eine zweite Begrenzung hat, die durch das metallisches Maschen-Material (3) gebildet wird.

2. Das medizinische Gerät gemäß Anspruch 1, wobei der Kanal im Ausmaß begrenzt ist, derart daß er nur teilweise entlang der Länge des metallisches Maschen-Materials (3) führt.

3. Das medizinische Gerät gemäß Anspruch 2, mit einer Vielzahl von Passagen zwischen dem Ballon (2) und dem metallisches Maschen-Material (3).

## Revendications

1. Dispositif médical, comprenant :
un cathéter central (4) avec une lumière (5), ledit cathéter central (4) ayant ladite lumière (5) en tant que passage central à travers lui ;
un ballonnet (2) disposé l'extérieur dudit cathéter central (4), le ballonnet pouvant être gonflé et dégonflé, avec un état gonflé au cours du déploiement, et avec un état dégonflé avant le déploiement ;
un matériau en treillis métallique (3) disposé à l'extérieur dudit ballonnet (2) et entourant ledit ballonnet (2),
le dispositif médical ayant un canal (6) disposé entre ledit matériau en treillis métallique (3) et ledit ballonnet (2), ledit canal (6) étant prévu pour recevoir un fil-guide de canal (30),
le dispositif médical étant **caractérisé en ce que** le matériau en treillis (3) est prémonté sur le ballonnet gonflable, le matériau en treillis (3) devant être expansé lorsque le ballonnet (2) est gonflé au cours du déploiement, et **en ce que** des ouvertures (7) à travers le matériau en treillis métallique (3) sont prévues dans le canal (6) en de nombreux emplacements le long du canal (6) de telle sorte que le fil-guide de canal (30) puisse passer dans le canal (6) et sortir à travers l'une des ouvertures (7),
le canal (6) ayant une paroi supplémentaire (62) située entre le matériau en treillis métallique (3) et le ballonnet (2), et
le canal ayant une première limite au niveau du ballonnet replié et ayant une deuxième limite au niveau du matériau en treillis métallique (3).

2. Dispositif médical selon la revendication 1, dans lequel le canal (6) a une étendue limitée de telle sorte qu'il passe seulement en partie le long de la longueur du matériau en treillis métallique (3).

3. Dispositif médical selon la revendication 2, ayant une pluralité de passages entre le ballonnet (2) et le matériau en treillis métallique (3).
